# EUROPÄISCHE PATENTSCHRIFT

(11) **EP 2 030 680 B1**
(45) Veröffentlichungstag und Bekanntmachung des Hinweises auf die Patenterteilung: **02.07.2014**
(21) Anmeldenummer: 08166458.3
(22) Anmeldetag: 10.06.2002
(51) Int. Cl.: B01J 23/46, B01J 37/18, C07C 31/26, C07C 29/141

(54) **Ruthenium-Katalysatoren auf einem SiO2-Träger für die katalytische Hydrierung von Sacchariden**
Ruthenium catalysts on a SiO2 carrier for the catalytic hydrogenation of saccharides
Catalyseurs au ruthénium sur un support SiO2 pour l'hydrogénisation catalytique de saccharides

(30) Priorität: 11.06.2001 DE 10128205
(43) Veröffentlichungstag der Anmeldung: 04.03.2009
(62) Teilanmeldung aus: 02748756.0
(73) Patentinhaber: BASF SE, 67056 Ludwigshafen (DE)
(72) Erfinder: Vanoppen, Dominic, 2950 Kapellen (BE); Maas-Brunner, Melanie, 68165 Mannheim (DE); Kammel, Ulrich, 67346 Speyer (DE); Arndt, Jan-Dirk, 68167 Mannheim (DE)

(56) Entgegenhaltungen:
- EP-A- 0 992 475
- US-A- 3 055 840
- US-A- 5 334 790
- HONG A J ET AL: "EFFECT OF SILICA SUPPORT ON RU-CU CLUSTER MORPHOLOGY AS DETERMINED BY CATALYTIC ACTIVITY" JOURNAL OF PHYSICAL CHEMISTRY, AMERICAN CHEMICAL SOCIETY, US, Bd. 91, Nr. 10, 7. Mai 1987 (1987-05-07), Seiten 2665-2671, XP000852586 ISSN: 0022-3654
- ZOU W ET AL: "PRETREATMENT CHEMISTRY IN THE PREPARATION OF SILICA-SUPPORTED PT, RU, AND PT-RU CATALYSTS: AN IN SITU UV DIFFUSE REFLECTANCE STUDY" JOURNAL OF CATALYSIS, ACADEMIC PRESS, DULUTH, MN, US, Bd. 133, 1992, Seiten 202-219, XP001106141 ISSN: 0021-9517

## Beschreibung

Die vorliegende Erfindung betrifft neue Ruthenium-Katalysatoren, und ein Verfahren zu ihrer Herstellung.

Die erfindungsgemäßen Katalysatoren eignen sich für die Verwendung zur katalytischen Hydrierung von Mono- und Disacchariden bei der Herstellung von Zuckeralkoholen, ausgenommen von Sorbit.

Die großtechnische Herstellung von Zuckeralkoholen erfolgt in der Regel durch katalytische Hydrierung entsprechender Mono- und Disaccharide (siehe H. Schiweck et al. "Sugar Alcohols" in Ullmann's Encyclopedia of Industrial Chemistry, 5th ed. on CD-ROM). Zu diesem Zweck wurden als Katalysatoren bislang in erster Linie NickelKatalysatoren, z.B. Nickel-Trägerkatalysatoren oder Raney-Nickel, eingesetzt. Verschiedentlich wurde auch über den Einsatz von Ruthenium-haltigen Katalysatoren für diesen Zweck berichtet. In der Regel handelt es sich bei Ruthenium-Katalysatoren um sogenannte Trägerkatalysatoren, die Ruthenium auf einem oxidischen oder organischen Träger wie Kohle enthalten.

So beschreiben die US 4,380,680, US 4,487,980, US 4,413,152 und die US 4,471,144 Katalysatoren für die Hydrierung von Kohlehydraten zu den entsprechenden Zuckeralkoholen, die Ruthenium auf einem unter hydrothermalen Bedingungen stabilen Trägermaterial enthalten. Als hydrothermale Trägermaterialien werden alpha-Aluminiumoxid (US 4,380,680), Titan(IV)oxid (US 4,487,980), mit Titan(IV)halogenid behandeltes Aluminiumoxid (US 4,413,152) und theta-Aluminiumoxid (US 4,471,144) vorgeschlagen.

Aus der US 4,503,274 sind Katalysatoren für die Hydrierung von Kohlehydraten zu den entsprechenden Zuckeralkoholen bekannt, die durch Imprägnieren eines unter hydrothermalen Bedingungen stabilen Trägers mit einer wässrigen Rutheniumhalogenid-Lösung und anschließendes Hydrieren des Feststoffs bei Temperaturen im Bereich von 100 bis 300°C hergestellt werden.

Die US 3,963,788 beschreibt Ruthenium-Katalysatoren für die Hydrierung von Kohlehydraten, in denen das Ruthenium mit einem speziellen Zeolithen auf Basis eines Alumosilikats geträgert wurde. Die US 3,963,789 schlägt als Träger für Ruthenium-Katalysatoren kristalline Alumosilkat-Tone, insbesondere Montmorillonit vor.

Die FR-A 2526782 beschreibt die Verwendung eines durch Umsetzung von Natriumchlorid und Ruthenium via Na₂RuCl₆ hergestellten Rutheniumchlorids zur Herstellung von auf Siliziumdioxid geträgerten Ruthenium-Katalysatoren für die Hydrierung von Mono- und Oligosacchariden.

US 5,334,790 beschreibt ein Verfahren zur partiellen Hydrierung polycydischer und monocyclischer aromatischer Kohlenwasserstoffe zu den entsprechenden Cycloolefinen unter Verwendung eines Katalysators, enthaltend Ruthenium immobilisiert auf einem oxidischen Kompositmaterial.

Hong et al., J. Phys. Chem. 1987, 91 (10), Seiten 2665-2671, beschreiben die Herstellung sowie die Hydriereigenschaften von monometallischen Ruthenium-Katalysatoren und bimetallischen Ruthenium-Kupfer-Katalysatoren basierend auf verschiedenen Kieselgel-Trägermaterialien.

Zou et al., Joumal of Catalysis 1992, 133, Seiten 202-219, beschreiben die Herstellung und die Oberflächenbeschaffenheit von Hydrierkatalysatoren, erhältlich durch die Absorption von Pt(NH₃)₄(NO₃)₂ oder Ru(NH₃)₆Cl₃ auf Kieselgel.

EP 0992475 beschreibt ein Verfahren zur Herstellung von Alkoholen durch katalytische Hydrierung von Aldehyden oder Ketonen unter Verwendung von Ruthenium-Katalysatoren, welche durch die Behandlung eines oxidischen Trägermaterials aus der Reihe TiO₂, SiO₂, ZrO₂, MgO, Mischoxide und Silikate mit Rutheniumchlorid erhalten werden.

US 3,055,840 beschreibt Ruthenium-haltige Katalysatoren zur Reduktion von Aldehyden und Ketonen, enthaltend 20 - 95 Gew.-% Ruthenium, bezogen auf die im Katalysator enthaltenen katalytisch aktiven Metalle, sowie ein weiteres Platinmetall, ausgewählt unter Platin, Rhodium und Palladium, immobilisiert auf einem festen Trägermaterial.

Die aus dem Stand der Technik bekannten Ruthenium-Katalysatoren weisen bei der Hydrierung von Kohlehydraten nur mässige Reaktivitäten auf mit der Folge, dass die erreichten Raum-Zeit-Ausbeuten an Zuckeralkoholen, bezogen auf den eingesetzten Katalysator gering sind. Angesichts der hohen Kosten für Ruthenium lässt daher die Wirtschaftlichkeit dieser Verfahren zu wünschen übrig. Zudem sind die Selektivitäten der Katalysatoren nicht ausreichend, so dass zusätzlicher Aufwand beim Isolieren der Wertprodukte erforderlich ist. Insbesondere wird häufig eine Epimerisierung der Hydroxygruppen beobachtet.

Der vorliegenden Erfindung liegt daher die Aufgabe zugrunde, neue Katalysatoren für die Herstellung von Zuckeralkoholen durch katalytische Hydrierung der entsprechenden Kohlehydrate bereitzustellen, die hohe Reaktivitäten aufweisen. Weiterhin sollten die Katalysatoren eine hohe Produkt-Selektivität aufweisen, nicht zuletzt im Hinblick auf eine kontinuierliche Ausgestaltung der Reduktion.

Diese Aufgabe wird gelöst durch einen Ruthenium-Katalysator, welcher Ruthenium in einer Menge von 0,2 bis 10 Gew.-% bezogen auf das Gewicht des Trägers enthält, erhältlich durch:
i) ein oder mehrfaches Behandeln eines Trägermaterials auf Basis von amorphem Siliziumdioxid, welches zu wenigstens 90 Gew.-% aus Siliziumdioxid besteht und weniger als 1 Gew.-% Aluminiumoxid, gerechnet als Al₂O₃, enthält, mit einer wässrigen Lösung einer Rutheniumverbindung, ausgewählt unter Ruthenium(III)nitrosylnitrat, Ruthenium(III)acetat, Natrium- und Kaliumruthenat(IV), wobei die wässrige Lösung, bezogen auf ihr Gesamtgewicht, weniger als 500 ppm Halogen enthält, und anschließendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C.
wobei man Schritt ii) unmittelbar im Anschluss an Schritt i) durchführt.

Die Erfindung betrifft somit diese Katalysatoren sowie das hier beschriebene Verfahren zu ihrer Herstellung. Die erfindungsgemäßen Katalysatoren zeichnen sich durch eine erhöhte Aktivität und hohe Produktselektivität bei der Hydrierung von Mono- und Oligosacchariden aus.

Es wird vermutet, dass die hohe Reaktivität der erfindungsgemäßen Katalysatoren auf die besonderes gute Verteilung des Rutheniums auf der Oberfläche des Trägermaterials und auf die weitgehende Abwesenheit von Halogen im Trägermaterial zurückgeführt werden kann. Herstellungsbedingt liegt das Ruthenium in den erfindungsgemäßen Katalysatoren als metallisches Ruthenium vor. Elektronenmikroskopische Untersuchungen (TEM) der erfindungsgemäßen Katalysatoren haben gezeigt, dass das Ruthenium auf dem Trägermaterial in atomar-dipserser Form und/oder in Form von Ruthenium-Partikeln vorliegt, die nahezu ausschliesslich, d.h. zu mehr als 90 %, vorzugsweise zu mehr als 95 %, bezogen auf die Anzahl der sichtbaren Partikel, als isolierte Partikel mit Durchmessern unterhalb 10 nm, insbesondere unterhalb 7 nm vorliegen. Mit anderen Worten, der Katalysator enthält im Wesentlichen keine, d.h. zu weniger als 10 %, insbesondere weniger als 5 % Ruthenium-Partikel und/oder Agglomerate von Rutheniumpartikeln mit Durchmessern oberhalb 10 nm. Durch die Verwendung halogenfreier Rutheniumprekursoren und Lösungsmittel bei der Herstellung liegt der Chlorgehalt der erfindungsgemäßen Katalysatoren zudem unterhalb 0,05 Gew.-% (< 500 ppm), bezogen auf das Gesamtgewicht des Katalysators.

Ein wesentlicher Aspekt der erfindungsgemäßen Katalysatoren ist die Verwendung eines Trägermaterials auf der Basis von amorphem Siliziumdioxid. Unter dem Begriff "amorph" versteht man in diesem Zusammenhang, dass der Anteil kristalliner Siliziumdioxid-Phasen weniger als 10 % des Trägermaterials ausmacht. Die zur Herstellung der erfindungsgemäßen Katalysatoren verwendeten Trägermaterialien können allerdings Überstrukturen aufweisen, die durch regelmässige Anordnung von Poren im Trägermaterial gebildet werden.

Als Trägermaterialien kommen grundsätzlich alle amorphen Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-% des Trägermaterials auch ein anderes oxidisches Material sein können, z.B. MgO, CaO, TiO₂, ZrO₂, Fe₂O₃ oder Alkalimetalloxid. Es versteht sich von selbst, dass das eingesetzte Trägermaterial ebenfalls halogenfrei ist, d. h. der Halogengehalt beträgt weniger als 500 ppm. Vorzugsweise enthält das Trägermaterial nicht mehr als 1 Gew.-% und insbesondere nicht mehr als 0,5 Gew.-% und insbesondere keine nachweisbaren Mengen (< 500 ppm) an Aluminiumoxid, gerechnet als Al₂O₃. In einer bevorzugten Ausführungsform verwendet man Trägermaterialien, die weniger als 500 ppm Fe₂O₃ enthalten. Der Anteil an Alkalimetalloxid resultiert in der Regel aus der Herstellung des Trägermaterials und kann bis zu 2 Gew.% betragen. Häufig beträgt er weniger als 1 Gew.-%. Geeignet sind auch Alkalimetalloxid-freie Träger (< 0,1 Gew.-%). Der Anteil an MgO, CaO, TiO₂ bzw. an ZrO₂ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (< 0,1 Gew.-%).

Bevorzugt sind Trägermaterialien, die eine spezifische Oberfläche im Bereich von 50 bis 700 m²/g, insbesondere im Bereich von 80 bis 600 m²/g und speziell im Bereich von 100 bis 600 m²/g aufweisen (BET-Oberfläche nach DIN 66131). Unter den pulverförmigen Trägermaterialien sind insbesondere solche bevorzugt, deren spezifische (BET) Oberfläche im Bereich von 200 bis 600 m²/g liegt. Bei Trägermaterial in Form von Formkörpern liegt die spezifische Oberfläche insbesondere im Bereich von 100 bis 300 m²/g.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 5th ed. on CD-ROM). Sie können sowohl natürlichen Ursprungs als auch künstlich hergestellt worden sein. Beispiele für geeignete amorphe Trägermaterialien auf Basis von Siliziumdioxid sind Kieselgur, Kieselgele, pyrogene Kieselsäure und Fällungskieselsäure. In einer bevorzugten Ausführungsform der Erfindung enthalten die Katalysatoren Kieselgele als Trägermaterialien.

Je nach Ausgestaltung der Hydrierverfahren, in denen die erfindungsgemäßen Katalysatoren eingesetzt werden, kann das Trägermaterial unterschiedliche Gestalt aufweisen. Sofern das Verfahren als Suspensionsverfahren ausgestaltet ist, wird man zur Herstellung der erfindungsgemäßen Katalysatoren üblicherweise das Trägermaterial in Form eines feinteiligen Pulvers einsetzen. Vorzugsweise weist das Pulver Teilchengrössen im Bereich von 1 bis 200 µm insbesondere 1 bis 100 µm auf. Bei Einsatz des Katalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 1 mm bis 25 mm. Häufig werden Katalysatorstränge mit Strangdurchmessern von 2 bis 5 mm und Stranglängen von 2 bis 25 mm eingesetzt.

Der Gehalt an Ruthenium in den erfindungsgemäßen Katalysatoren beträgt wenigstens 0,2 Gew.-% und überschreite einen Wert von 10 Gew.-%, jeweils bezogen auf das Gewicht des Trägermaterials, nicht. Vorzugsweise liegt der Gehalt an Ruthenium im Bereich von 0,2 bis 7 Gew.-% und insbesondere im Bereich von 0,4 bis 5 Gew.-%.

Zur Herstellung der erfindungsgemäßen Ruthenium-Katalysatoren wird das Trägermaterial zunächst mit einer halogenfreien wässrigen Lösung einer niedermolekularen Rutheniumverbindung, im Folgenden als (Ruthenium)prekursor bezeichnet, in einer Weise behandelt, dass die gewünschte Menge an Ruthenium vom Trägermaterial aufgenommen wird. Dieser Schritt wird im Folgenden auch als Tränken bezeichnet. Anschliessend wird der so behandelte Träger bei den oben angegebenen Temperaturen getrocknet. Gegebenenfalls wird dann der so erhaltene Feststoff erneut mit der wässrigen Lösung des Rutheniumprekursors behandelt und erneut getrocknet. Dieser Vorgang wird so oft wiederholt, bis die vom Trägermaterial aufgenommene Menge an Rutheniumverbindung dem gewünschten Rutheniumgehalt im Katalysator entspricht.

Das Behandeln bzw. Tränken des Trägermaterials kann in unterschiedlicher Weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in der wässrigen Lösung des Rutheniumprekursors suspendieren und nach einer gewissen Zeit vom wässrigen Überstand abfiltrieren. Über die aufgenommene Flüssigkeitsmenge und die Ruthenium-Konzentration der Lösung kann dann der Rutheniumgehalt des Katalysators in einfacher Weise gesteuert werden. Das Tränken des Trägermaterials kann beispielsweise auch dadurch erfolgen, dass man den Träger mit einer definierten Menge der wässrigen Lösung des Rutheniumprekursors behandelt, die der maximalen Flüssigkeitsmenge entspricht, die das Trägermaterial aufnehmen kann. Zu diesem Zweck kann man beispielsweise das Trägermaterial mit der Flüssigkeitsmenge besprühen. Geeignete Apparaturen hierfür sind die zum Vermengen von Flüssigkeiten mit Feststoffen üblicherweise verwendeten Apparate (siehe Vauck/Müller, Grundoperationen chemischer Verfahrenstechnik, 10. Auflage, Deutscher Verlag für Grundstoffindustrie, 1994, S. 405 ff.) beispielsweise Taumeltrockner, Tränktrommeln, Trommelmischer, Schaufelmischer und dergleichen. Monolithische Träger werden üblicherweise mit den wässrigen Lösungen des Rutheniumprekursors gespült.

Die zum Tränken eingesetzten wässrigen Lösungen sind erfindungsgemäß halogenfrei, d.h. sie enthalten kein oder weniger als 500 ppm Halogen. Als Rutheniumprekursoren werden daher nur solche Rutheniumverbindungen eingesetzt, die kein chemisch gebundenes Halogen enthalten und die in dem wässrigen Lösungsmittel hinreichend löslich sind. Diese sind Ruthenium(III)nitrosylnitrat (Ru(NO)(NO₃)₃), Ruthenium(III)acetat sowie die Alkalimetallruthenate(IV) Natrium- und Kaliumruthenat(IV).

Der Begriff "wässrig" bezeichnet hier Wasser sowie Mischungen von Wasser mit bis zu 50 Vol.-%, vorzugsweise nicht mehr als 30 Vol.-% und insbesondere nicht mehr als 10 Vol.-% eines oder mehrerer mit Wasser mischbarer organischer Lösungsmittel, z.B. Mischungen von Wasser mit C₁-C₄-Alkanolen wie Methanol, Ethanol, n- oder Isopropanol. Häufig setzt man Wasser als alleiniges Lösungsmittel ein. Das wässrige Lösungsmittel wird häufig zusätzlich wenigstens eine halogenfreie Säure, z.B. Salpetersäure, Schwefelsäure, Phosphorsäure oder Essigsäure, vorzugsweise eine halogenfreie Mineralsäure, zur Stabilisierung des Rutheniumprekursors in der Lösung enthalten. In vielen Fällen setzt man daher eine mit Wasser verdünnte, halogenfreie Mineralsäure, z. B. verdünnte bis halbkonzentrierte Salpetersäure als Lösungsmittel für den Rutheniumprekursor ein. Die Konzentration des Rutheniumprekursors in den wässrigen Lösungen richtet sich naturgemäss nach der aufzubringenden Menge an Rutheniumprekursor und der Aufnahmekapazität des Trägermaterials für die wässrige Lösung und liegt in der Regel im Bereich von 0,1 bis 20 Gew.-%.

Das Trocknen kann nach den üblichen Verfahren der Feststofftrocknung unter Einhaltung der obengenannten Temperaturen erfolgen. Die Einhaltung der erfindungsgemäßen Obergrenze der Trocknungstemperaturen ist für die Qualität, d.h. die Aktivität des Katalysators wichtig. Ein Überschreiten der oben angegebenen Trocknungstemperaturen führt zu einem deutlichen Verlust an Aktivität. Ein Kalzinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus.

Die Trocknung des in mit dem Rutheniumprekursors getränkten Feststoff erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäss von dem gewünschten Grad der Trocknung und der Trocknungstemperatur ab und liegt in der Regel im Bereich von 2 h bis 30 h, vorzugsweise im Bereich von 4 h bis 15 h.

Vorzugsweise führt man die Trocknung des behandelten Trägermaterials soweit, dass der Gehalt an Wasser bzw. an flüchtigen Lösungsmittelbestandteilen vor der Reduktion ii) weniger als 5 Gew.-%, insbesondere nicht mehr als 2 Gew.-% und besonders bevorzugt nicht mehr als 1 Gew.-%, bezogen auf das Gesamtgewicht des Feststoffs ausmacht. Die angegebenen Gewichtsanteile beziehen sich auf den Gewichtsverlust des Feststoffs, bestimmt bei einer Temperatur von 300 °C, einem Druck von 1 bar und einer Dauer von 10 min. Auf diese Weise kann die Aktivität der erfindungsgemäßen Katalysatoren weiter gesteigert werden.

Vorzugsweise erfolgt das Trocknen unter Bewegen des mit der Prekursor-Lösung behandelten Feststoffs, beispielsweise durch Trocknen des Feststoffs in einem Drehrohrofen oder einem Drehkugelofen. Auf diese Weise kann die Aktivität der erfindungsgemäßen Katalysatoren weiter gesteigert werden.

Die Überführung des nach dem Trocknen erhaltenen Feststoffs in seine katalytisch aktive Form erfolgt erfindungsgemäß durch Hydrieren des Feststoffs bei den oben angegebenen Temperaturen in an sich bekannter Weise.

Zu diesem Zweck bringt man das Trägermaterial bei den oben angegebenen Temperaturen mit Wasserstoff oder einer Mischung aus Wasserstoff und einem Inertgas in Kontakt. Der Wasserstoffpartialdruck ist für das Ergebnis der Reduktion von untergeordneter Bedeutung und kann im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt das Hydrieren unter Bewegen des in i) erhaltenen Feststoffs, beispielsweise durch Hydrieren des Feststoffs in einem Drehrohrofen oder einem Drehkugelofen. Auf diese Weise kann die Aktivität der erfindungsgemäßen Katalysatoren weiter gesteigert werden.

Im Anschluss an die Hydrierung kann der Katalysator zur Verbesserung der Handhabbarkeit in bekannter Weise passiviert werden, z.B. indem man den Katalysator kurzfristig mit einem Sauerstoff-haltigen Gas, z.B. Luft, vorzugsweise jedoch mit einer 1 bis 10 Vol.% Sauerstoff enthaltenden Inertgasmischung behandelt.

Die erfindungsgemässen Katalysatoren können als Hydrierkatalysatoren für eine Vielzahl von Hydrierreaktionen eingesetzt werden, welche die Hydrierung von C=C-, C=O- und C=N-Doppelbindungen sowie die Hydrierung von C≡C- und C≡N-Dreifachbindungen umfassen.

In besonderem Maße eignen sich die erfindungsgemässen Katalysatoren zur Hydrierung der Carbonylfunktion von Mono- und Oligosacchariden. Sie zeichnen sich gegenüber diesen Substraten zum einen durch sehr hohe Aktivitäten aus, so dass hohe Raum-Zeit-Ausbeuten, bezogen auf den eingesetzten Katalysator, insbesondere auf eingesetztes Ruthenium erreicht werden. Zudem werden die entsprechenden Zuckeralkohole in hohen Ausbeuten erhalten. Weiterhin ist die Produktselektivität hoch, d.h. das Auftreten von Nebenreaktion wie Epimerisierung, Decarbonylierung, Oligomerisierung und dergleichen, die zu Ausbeuteverlusten führen, ist geringer als bei den Ruthenium-Katalysatoren des Standes der Technik. Durch die erhöhte Produktselektivität verringert sich ausserdem der Aufwand für die Isolierung des gewünschten Hydrierungsprodukts. Weiterhin vereinfacht sich hierdurch eine kontinuierliche Durchführung der Reaktion. Ausserdem zeichnen sich die erfindungsgemässen Katalysatoren durch hohe Standzeiten, selbst unter den aggressiven Bedingungen einer Hydrierung in einem wässrigen Reaktionsmedium aus, d.h. ein Aktivitätsverlust der erfindungsgemäßen Katalysatoren wird auch nach längerer Einsatzdauer im erfindungsgemäßen Hydrierverfahren, z.B. nach 1100 h, nicht oder nicht in nennenswertem Maße beobachtet.

Selbstverständlich können die in diesem Verfahren eingesetzten Katalysatoren bei nachlassender Aktivität nach den für Edelmetallkatalysatoren wie Rutheniumkatalysatoren üblichen, dem Fachmann bekannten Methoden regeneriert werden. Hier sind z. B. die Behandlung des Katalysators mit Sauerstoff wie in der BE 882279 beschrieben, die Behandlung mit verdünnten, halogenfreien Mineralsäuren, wie in der US 4,072,628 beschrieben, oder die Behandlung mit Wasserstoffperoxid, z. B. in Form wässriger Lösungen mit einem Gehalt von 0,1 bis 35 Gew.-%, oder die Behandlung mit anderen oxidierenden Substanzen, vorzugsweise in Form halogenfreier Lösungen zu nennen. Üblicherweise wird man den Katalysator nach der Reaktivierung und vor dem erneuten Einsatz mit einem Lösungsmittel, z. B. Wasser, spült.

Die erfindungsgemäßen Katalysatoren sind zur Herstellung von Zuckeralkohlen durch katalytische Hydrierung der korrespondierenden Mono- und Oligosaccharide, insbesondere der Mono- und Disaccharide, in flüssiger Phase an einem heterogenen Ruthenium-Katalysator, geeignet.

Geeignete Saccharide umfassen grundsätzlich alle bekannten Tetrosen, Pentosen, Hexosen und Heptosen und zwar sowohl Aldosen als auch Ketosen sowie deren Di- und Oligosaccaride, wobei Glucose, Fructose, Gulose und Saccharose ausgenommen sind, da sie bei Hydrierung Sorbit liefern. Zu den Monosacchariden, die in diesem Verfahren eingesetzt werden können, zählen beispielsweise: Erythrose, Threose, Ribose, Arabinose, Xylose, Lyxose, Allose, Altrose, Mannose, Gulose, Idose, Galactose, Talose, Erythrulose, Ribulose, Xylulose, Psicose und Tagatose und zwar sowohl die D-Form als auch die L-Form. Beispiele für Disacharide sind: Maltose, Isomaltose, Lactose, Cellobiose und Melobiose. Die Mono- und Oligosaccharide können als solche oder als Mischungen eingesetzt werden, wobei man vorzugsweise die Edukte in Reinform einsetzt.

Als geeignete Mono- und Oligosaccharide für das Hydrierverfahren sind inbesondere die Monosaccharide Mannose für die Herstellung von Mannit, Galactose für die Herstellung von Dulcit (Galaktit) und Xylose für die Herstellung von Xylit, vorzugsweise die D-Form der Monosaccharide, sowie die Disaccharide Maltose für die Herstellung von Maltit, Isomaltulose (Palatinose) für die Herstellung von Isomaltit und Lactose für die Herstellung von Lactit zu nennen. Aber auch die anderen genannten Mono- und Oligosaccharide können in Gegenwart der erfindungsgemäßen Ruthenium-Katalysatoren zu den korrespondierenden Zuckeralkoholen hydriert werden. Dabei führt die Hydrierung von Aldosen zu Zuckeralkoholen, die hinsichtlich der OH-Gruppen die gleiche Konfiguration wie der eingesetzte Zucker aufweisen, und die Hydrierung von Furanosen in der Regel zu Gemischen zweier diastereomerer Zuckeralkohole, die sich nur in der Konfiguration des C-Atoms unterscheiden, welches in der Furanose die Carbonylfunktion trägt. Die Isolierung des jeweiligen reinen Zuckeralkohols aus dieser Mischung ist in der Regel ohne Probleme möglich.

Die Hydrierung erfolgt vorzugsweise durch Hydrieren einer Lösung des jeweiligen Mono- oder Oligosaccharids in einem wässrigen Lösungmittel. Der Begriff "wässrig" ist hierbei in der oben definierten Weise zu verstehen.

Zweckmäßigerweise wird Wasser als alleiniges Lösungsmittel verwendet, das gegebenenfalls geringe Mengen einer vorzugsweise halogenfreien Säure zur Einstellung des pH-Wertes enthält. Insbesondere setzt man das Mono- oder das Oligosacharid als wässrige Lösung ein, die einen pH-Wert im Bereich von 4 bis 10, und speziell im Bereich von 5 bis 7 aufweist.

Die Konzentration an Edukten in der flüssigen Phase kann grundsätzlich frei gewählt werden und liegt häufig im Bereich von 10 bis 80 Gew.-% und vorzugsweise im Bereich von 15 bis 50 Gew.-%, bezogen auf das Gesamtgewicht der Lösung.

Die eigentliche Hydrierung unter Verwendung der erfindungsgemäßen Katalysatoren erfolgt üblicherweise in Analogie zu den bekannten Hydrierverfahren für die Herstellung von Zuckeralkoholen, wie sie im eingangs genannten Stand der Technik beschrieben werden. Hierzu wird die flüssige, das Edukt enthaltende Phase mit dem Katalysator in Gegenwart von Wasserstoff in Kontakt gebracht. Der Katalysator kann dabei sowohl in der zu hydrierenden flüssigen Phase suspendiert werden (Suspensionsfahrweise) oder man führt die flüssige Phase über ein Katalysator-Fließbett (Fließbett-Fahrweise) oder ein Katalysator-Festbett (Festbettfahrweise). Die Hydrierung kann dabei sowohl kontinuierlich als auch diskontinuierlich ausgestaltet werden. Vorzugsweise führt man das erfindungsgemäße Verfahren in Rieselbettreaktoren nach der Festbettfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Suspensionsfahrweise als auch zur Hydrierung am Katalysatorfestbett sind aus dem Stand der Technik bekannt, z.B. aus Ullmanns Enzyklopädie der Technischen Chemie, 4. Auflage, Band 13, S. 135 ff. sowie aus P. N. Rylander, "Hydrogenation and Dehydrogenation" in Ullmann's Encyclopedia of Industrial Chemistry, 5th ed. on CD-ROM bekannt.

In der Regel führt man die Hydrierung bei erhöhtem Wasserstoff-Druck, z.B. bei einem Wasserstoffpartialdruck von wenigstens 10 bar, vorzugsweise wenigstens 20 bar und insbesondere wenigstens 40 bar durch. In der Regel wird der Wasserstoffpartialdruck einen Wert von 500 bar, insbesondere 350 bar nicht überschreiten. Besonders bevorzugt liegt der Wasserstoffpartialdruck im Bereich von 40 bis 200 bar. Die Reaktionstemperaturen betragen in der Regel wenigstens 40°C und werden häufig einen Wert von 250°C nicht überschreiten. Insbesondere führt man das Hydrierverfahren bei Temperaturen im Bereich von 80 bis 150°C durch.

Aufgrund der hohen Katalysatoraktivität benötigt man vergleichsweise geringe Mengen an Katalysator bezogen auf das eingesetzte Edukt. So wird man bei der diskontinuierlchen Suspensionsfahrweise in der Regel weniger als 1 mol-%, z.B. 10⁻³ mol-% bis 0,5 mol-% Ruthenium, bezogen auf 1 mol Zucker einsetzen. Bei kontinuierlicher Ausgestaltung des Hydrierverfahrens wird man üblicherweise das zu hydrierende Edukt in einer Menge von 0,02 bis 2 kg/(l (Katalysator)*h) und vorzugsweise in einer Menge von 0,07 bis 0,7 kg/(l (Katalysator)*h) über den Katalysator führen.

Die folgenden Beispiele dienen der nähren Erläuterung der Erfindung:

### I Herstellung der erfindungsgemäßen Katalysatoren

1. Vorschrift A: Pulverförmiger, halogenfreier Katalysator, nicht kalziniert.
Eine definierte Menge des jeweiligen Trägermaterials wurde mit der maximalen Menge einer Lösung von Ruthenium(III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden. Die Konzentration der Lösung wurde jeweils so bemessen, dass die gewünschte Konzentration an Ruthenium im Trägermaterial resultierte.
Anschliessend wurde der so erhaltene Feststoff 13 h bei 120°C in einem Trockenschrank getrocknet. Der Restwassergehalt lag unter 1 Gew.-%.
Der so erhaltene Feststoff wurde in einem Reaktionsrohr 4 h bei 300°C im Wasserstoffstrom bei Normaldruck reduziert. Nach Abkühlen und Inertisieren mit Stickstoff wurde der Katalysator durch Überleiten von 5 Vol.-% Sauerstoff in Stickstoff über einen Zeitraum von 2 h passiviert.
2. Vorschrift B: Pulverförmiger, halogenfreier Katalysator, bewegt getrocknet, nicht kalziniert.
Die Herstellung erfolgte analog Vorschrift A, jedoch wurden Trocknung in einem Drehkugelofen durchgeführt. Der Restwassergehalt lag unter 1 Gew.-%.
3. Vorschrift C: Pulverförmiger, halogenfreier Katalysator, kalziniert.
Die Herstellung erfolgte analog Vorschrift B, jedoch wurde der nach dem Trocknen erhaltene Feststoff vor der Hydrierung 4 h auf 400°C im Luftstrom erhitzt.
4. Vorschrift D: Pulverförmiger, halogenhaltiger Katalysator, nicht kalziniert.
Die Herstellung erfolgte analog Vorschrift B, jedoch wurde anstelle von Ruthenium(III)nitrosylnitrat Ruthenium(III)chlorid eingesetzt.
5. Vorschrift E: Strangförmiger, halogenfreier Katalysator, nicht kalziniert.
Eine definierte Menge von zylindrischen Trägermaterial-Strängen (Durchmesser 4 mm, Länge 3 bis 10 mm) wurde mit der maximalen Menge einer Lösung von Ruthenium(III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden.
Die Konzentration der Lösung wurde jeweils so bemessen, dass die gewünschte Konzentration an Ruthenium im Trägermaterial resultierte.
Anschliessend wurden die so erhaltenen, getränkten Stränge 13 h bei 120°C in einer Drehkugelofen getrocknet. Der Restwassergehalt lag unter 1 Gew.-%.
Die so erhaltenen, getrockneten Stränge wurden in einem Drehkugelofen 4 h bei 300°C im Wasserstoffstrom bei Normaldruck reduziert. Nach Abkühlen und Inertisieren mit Stickstoff wurde der so erhaltene Katalysator durch Überleiten von 5 Vol.-% Sauerstoff in Stickstoff über einen Zeitraum von 2 h passiviert.

**Tabelle 1: Katalysatoren**

| Katalysator Nr. | Rutheniumgehalt [Gew.-%] | Vorschrift | Träger |
|---|---|---|---|
| K1 | 5 | B | SiO₂ Pulver¹⁾ |
| K2 (V) | 5 | D | SiO₂ Pulver¹⁾ |
| K3 | 5 | A | SiO₂ Pulver¹⁾ |
| K4 (V) | 5 | C | SiO₂ Pulver¹⁾ |
| K5 (V) | 5 | B | α-Al₂O₃ Pulver²⁾ |
| K6 (V) | 5 | B | θ-Al₂O₃ Pulver³⁾ |
| K7 (V) | 5 | B | TiO₂ Pulver⁴⁾ |
| K8 | 1 | E | SiO₂ Stränge⁵⁾ |

### V Vergleichskatalysator

1) Kieselgel-Pulver mit einem SiO₂-Gehalt > 99,95 Gew.-%,
   einer spezifischen BET-Oberfläche von 523 m²/g,
   einer Wasseraufnahme von 1,4 ml/g,
   einem Porenvolumen von 0,75 ml/g (ermittelt durch Stickstoffporometrie nach DIN 66134),
   einer definierten Porengröße von 60 Å
   einer Teilchengrösse von 63 bis 200 µm;
2) alpha-Aluminiumoxid-Pulver mit einem Al₂O₃-Gehalt > 99,95 Gew.-%,
   einer spezifischen BET-Oberfläche von 7 m²/g,
   einer Wasseraufnahme von 0,84 ml/g,
   einer Teilchengrösse < 100 µm;
3) theta-Aluminiumoxid-Pulver mit einem Al₂O₃-Gehalt > 99,95 Gew.-%,
   einer spezifischen BET-Oberfläche von 80 m²/g,
   einer Wasseraufnahme von 1,05 ml/g,
   einem Porenvolumen von 0,67 ml/g (DIN 66134),
   einer Teilchengrösse < 100 µm;
4) Titandioxid-Pulver mit einem TiO₂-Gehalt > 99,9 Gew.-%,
   einer spezifischen BET-Oberfläche von 325 m²/g,
   einer Wasseraufnahme von 0,84 ml/g,
   einer Teilchengrösse < 63 µm;
5) Kieselgel-Stränge (d 4 mm, I 1 bis 10 mm) aus Kieselgel mit einem SiO₂-Gehalt > 99,5 Gew.-% (0,3 Gew.-% Na₂O),
   einer spezifischen BET-Oberfläche von 169 m²/g,
   einer Wasseraufnahme von 0,95 ml/g,
   einem Porenvolumen von 0,7 ml/g (DIN 66134).

### II. Hydrierung von Xylose in Suspensionsfahrweise (Beispiele 1 und 2, Vergleichsbeispiele V1 bis V5)

### Allgemeine Hydriervorschrift:

In einem 2,5 l Autoklaven mit Rührer, Vorrichtungen zur Probenentnahme und einer Druckhaltung für Wasserstoff wurden 1200 ml einer 30 Gew.-% Lösung von Xylose in Wasser zusammen mit 3 g des jeweiligen Katalysators vorgelegt. Der Autoklav wurde mit Stickstoff inertisiert. Anschließend presste man 100 bar Wasserstoff auf und erwärmte den Autoklaven auf 90°C. Während der Reaktion wurde mit 1000 U/min gerührt. Zur Ermittlung des Umsatzes wurden während der Reaktion regelmäßig Proben entnommen und mittels HPLC der Gehalt an Xylose, Xylit und sonstigen Produkten bestimmt. Die Umsetzung wurde spätestens nach 10 h abgebrochen. In Tabelle 2 ist die Zeitdauer angegeben, die zur Erreichung eines maximalen Ausbeute erforderlich ist. Ausserdem ist die Selektivität hinsichtlich der Bildung von Xylit angegeben, die mit einer Genauigkeit von etwa 0,5 % (absolut) bestimmt werden konnte.

**Tabelle 2:**

| Beispiel | Kat. Nr. | Träger | t-max. [h] | Umsatz [%] | Selektivität [%] |
|---|---|---|---|---|---|
| 1 | K1 | SiO₂ Pulver | 3 | 99,85 | > 98,5 |
| V1 | K2 (V) | SiO₂ Pulver | 7 | 99,85 | > 98,5 |
| 2 | K3 | SiO₂ Pulver | 5 | 99,95 | > 98,5 |
| V2 | K4 (V) | SiO₂ Pulver | 10 | 99,96 | > 98,5 |
| V3 | K5 (V) | α-Al₂O₃ Pulver | 10 | 94,15 | 98,5 |
| V4 | K6 (V) | θ-Al₂O₃ Pulver | 10 | 99,53 | > 98,5 |
| V5 | K7 (V) | TiO₂ Pulver | 10 | 76,84 | 98,2 |

### III Hydrierung von Mannose, Maltose und Lactose in Suspensionsfahrweise (Beispiele 3, 4 und 5)

Analog der unter II angegebenen allgemeinen Hydriervorschrift wurden wurden 1200 ml einer 30 Gew.-% Lösung des jeweiligen Mono- bzw. Disaccharids in Wasser zusammen mit 3 g des jeweiligen Katalysators bei 50 bar Wasserstoff und einer Temperatur von 120°C hydriert. Umsatz und Selekivität wurden wie unter II beschrieben mittels HPLC ermittelt. In Tabelle 3 ist die Zeitdauer angegeben, die zur Erreichung eines maximalen Umsatzes (> 99,8%) erforderlich ist. Ausserdem ist die Selektivität hinsichtlich der Bildung des gewünschten Zuckeralkohols angegeben.

**Tabelle 3:**

| Beispiel | Kat.Nr. | Edukt | Produkt | t-max. [h] | Umsatz [%] | Selektivität [%] |
|---|---|---|---|---|---|---|
| 3 | K1 | Mannose | Mannit | 2 | >99,8 | 96 |
| 4 | K1 | Maltose | Maltit | 1 | >99,8 | 69 |
| 5 | K1 | Lactose | Lactit | 3 | >99,8 | 87 |

### III Hydrierung von Xylose und Lactose am Katalysatorfestbett (Beispiele 6 und 7)

Als Reaktor diente ein beheizbares Reaktionsrohr aus Edelstahl, das mit Katalysator K8 gefüllt war. Die Reaktionsanordnung wies eine Zulaufpumpe für die Edukte, eine Kreislaufpumpe, Vorrichtungen für die Probenentnahme sowie einen Abscheider mit Standregelung und Abgasregelung auf.

In dieser Reaktionsanordnung wurde eine 30 gew.-%ige Lösung des jeweiligen Mono- bzw. Disaccharids bei einer Temperatur von 100 °C und einem Wasserstoffdruck von 50 bar mit einer Geschwindigkeit von 50 ml/(g(Katalysator)*h) im Kreis gefahren und währenddessen mittels der unter II beschriebenen Analytik die Abnahme des Edukts, die Zunahme des Produkte und die Bildung von Nebenprodukten bestimmt. Bei erreichen eines Umsatzes von 99,4 % wurde die Umsetzung abgebrochen. Die zur Erreichung des maximalen Umsatzes erforderliche Kontaktzeit ist in Tabelle 4 zusammen mit der Selektivität angegeben. Kontaktzeit = Vol.(Lösung)/Vol.(Reaktionsrohr)*Reaktionszeit

**Tabelle 4:**

| Beispiel | Edukt | Produkt | Kontaktzeit. [h] | Selektivität [%] |
|---|---|---|---|---|
| 6 | Xylose | Xylit | 0,81 | 97,2 |
| 7 | Lactose | Lactit | 1,0 | 94,1 |

## Patentansprüche

1. Ruthenium-Katalysator, welcher Ruthenium in einer Menge von 0,2 bis 10 Gew.-% bezogen auf das Gewicht des Trägers enthält, erhältlich durch:
i) ein oder mehrfaches Behandeln eines Trägermaterials auf Basis von amorphem Siliziumdioxid, welches zu wenigstens 90 Gew.-% aus Siliziumdioxid besteht und weniger als 1 Gew.-% Aluminiumoxid, gerechnet als Al₂O₃, enthält, mit einer wässrigen Lösung einer Rutheniumverbindung, ausgewählt unter Ruthenium(III)nitrosylnitrat, Ruthenium(III)acetat, Natrium- und Kaliumruthenat(IV), wobei die wässrige Lösung, bezogen auf ihr Gesamtgewicht, weniger als 500 ppm Halogen enthält, und anschließendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C,
wobei man Schritt ii) unmittelbar im Anschluss an Schritt i) durchführt.

2. Ruthenium-Katalysator nach Anspruch 1, worin der Träger auf Basis von amorphem Siliziumdioxid eine BET-Oberfläche im Bereich von 50 bis 700 m²/g aufweist.

3. Ruthenium-Katalysator nach einem der vorhergehenden Ansprüche, wobei der zur Reduktion ii) eingesetzte, aus i) erhaltene Feststoff einen Wassergehalt von weniger 5 Gew.-%, bezogen auf das Gesamtgewicht des Feststoffs aufweist.

4. Ruthenium-Katalysator nach einem der vorhergehenden Ansprüche, wobei das Trocknen in Schritt i) unter Bewegen des behandelten Trägermaterials erfolgt.

5. Verfahren zur Herstellung von Ruthenium-Katalysator nach einem der vorhergehenden Ansprüche, **dadurch gekennzeichnet, dass** es die folgenden Schritte umfasst:
i) ein oder mehrfaches Behandeln eines Trägermaterials auf Basis von amorphem Siliziumdioxid, welches zu wenigstens 90 Gew.-% aus Siliziumdioxid besteht und weniger als 1 Gew.-% Aluminiumoxid, gerechnet als Al₂O₃, enthält, mit einer wässrigen Lösung einer Rutheniumverbindung, ausgewählt unter Ruthenium(III)nitrosylnitrat, Ruthenium(III)acetat, Natrium- und Kaliumruthenat(IV), wobei die wässrige Lösung, bezogen auf ihr Gesamtgewicht, weniger als 500 ppm Halogen enthält, und anschließendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C,
wobei man Schritt ii) unmittelbar im Anschluss an Schritt i) durchführt.

## Claims

1. A ruthenium catalyst which comprises from 0.2 to 10% by weight of ruthenium, based on the weight of the support, obtainable by:
i) treating a support material based on amorphous silicon dioxide which comprises at least 90% by weight of silicon dioxide and less than 1% by weight of aluminum oxide, calculated as Al₂O₃, one or more times with an aqueous solution of a ruthenium compound, selected from among ruthenium(III) nitrosyl nitrate, ruthenium(III) acetate, sodium ruthenate(IV) and potassium ruthenate(IV), where the aqueous solution, based on its total weight, comprises less than 500 ppm of halogen, and subsequently drying the treated support material at below 200°C,
ii) reducing the solid obtained in i) by means of hydrogen at from 100 to 350°C,
where step ii) is carried out directly after step i).

2. A ruthenium catalyst according to claim 1, wherein the support based on amorphous silicon dioxide has a BET surface area in the range from 50 to 700 m²/g.

3. A ruthenium catalyst according to either of the preceding claims, wherein the solid which is obtained from i) and is used for the reduction ii) has a water content of less than 5% by weight, based on the total weight of the solid.

4. A ruthenium catalyst according to any of the preceding claims, wherein drying in step i) is carried out with the treated support material being kept in motion.

5. A process for producing a ruthenium catalyst according to any of the preceding claims, which comprises the following steps:
i) treating a support material based on amorphous silicon dioxide which comprises at least 90% by weight of silicon dioxide and less than 1% by weight of aluminum oxide, calculated as Al₂O₃, one or more times with an aqueous solution of a ruthenium compound, selected from among ruthenium(III) nitrosyl nitrate, ruthenium(III) acetate, sodium ruthenate(IV) and potassium ruthenate(IV), where the aqueous solution, based on its total weight, comprises less than 500 ppm of halogen, and subsequently drying the treated support material at below 200°C,
ii) reducing the solid obtained in i) by means of hydrogen at from 100 to 350°C,
where step ii) is carried out directly after step i).

## Revendications

1. Catalyseur au ruthénium, qui contient du ruthénium en une quantité de 0,2 à 10 % en poids par rapport au poids du support, pouvant être obtenu par :
i) le traitement à une ou plusieurs reprises d'un matériau support à base de dioxyde de silicium amorphe, qui est constitué d'au moins 90 % en poids de dioxyde de silicium et qui contient moins de 1 % en poids d'oxyde d'aluminium, calculé en tant qu'Al₂O₃, avec une solution aqueuse d'un composé de ruthénium choisi parmi le nitrate de nitrosyle de ruthénium (III), l'acétate de ruthénium (III), le ruthénate de sodium et de potassium (IV), la solution aqueuse contenant, par rapport à son poids total, moins de 500 ppm d'halogène, puis le séchage du matériau support traité à une température inférieure à 200 °C,
ii) la réduction du solide obtenu en i) avec de l'hydrogène à une température dans la plage allant de 100 à 350 °C,
l'étape ii) étant réalisée directement après l'étape i) .

2. Catalyseur au ruthénium selon la revendication 1, dans lequel le support à base de dioxyde de silicium amorphe présente une surface BET dans la plage allant de 50 à 700 m²/g.

3. Catalyseur au ruthénium selon l'une quelconque des revendications précédentes, dans lequel le solide obtenu en i) utilisé pour la réduction ii) présente une teneur en eau inférieure à 5 % en poids, par rapport au poids total du solide.

4. Catalyseur au ruthénium selon l'une quelconque des revendications précédentes, dans lequel le séchage à l'étape i) a lieu avec déplacement du matériau support traité.

5. Procédé de fabrication d'un catalyseur au ruthénium selon l'une quelconque des revendications précédentes, **caractérisé en ce qu'**il comprend les étapes suivantes :
i) le traitement à une ou plusieurs reprises d'un matériau support à base de dioxyde de silicium amorphe, qui est constitué d'au moins 90 % en poids de dioxyde de silicium et qui contient moins de 1 % en poids d'oxyde d'aluminium, calculé en tant qu'Al₂O₃, avec une solution aqueuse d'un composé de ruthénium choisi parmi le nitrate de nitrosyle de ruthénium (III), l'acétate de ruthénium (III), le ruthénate de sodium et de potassium (IV), la solution aqueuse contenant, par rapport à son poids total, moins de 500 ppm d'halogène, puis le séchage du matériau support traité à une température inférieure à 200 °C,
ii) la réduction du solide obtenu en i) avec de l'hydrogène à une température dans la plage allant de 100 à 350 °C,
l'étape ii) étant réalisée directement après l'étape i) .
